# EUROPEAN PATENT SPECIFICATION

(11) **EP 2 448 909 B1**
(45) Date of publication and mention of the grant of the patent: **01.05.2013**
(21) Application number: 10736978.7
(22) Date of filing: 28.06.2010
(51) Int. Cl.: C07C 209/62, C07C 211/35

(54) **Method of preparing 1-amino-1,3,3,5,5-pentamethylcyclohexane**
Verfahren zur Herstellung von 1-Amino-1, 3,3,5,5-Pentamethylcyclohexan
Procédé de préparation de 1-amino-1, 3,3,5,5-pentaméthylcyclohexane

(30) Priority: 29.06.2009 EP 09008465; 29.06.2009 US 269782 P
(43) Date of publication of application: 09.05.2012
(73) Proprietor: Merz Pharma GmbH & Co. KGaA, 60318 Frankfurt Main (DE)
(72) Inventor: KOLLER, Herbert, A-1100 Wien (AT); PYERIN, Michael, 2345 Brunn am Gebirge (AT)
(74) Representative: Ricker, Mathias
(86) International application number: PCT/EP2010/003924
(87) International publication number: WO 2011/000541

(56) References cited:
- JIRGENSONS A ET AL: "A Practical Synthesis of tert-Alkylamines via the Ritter Reaction with Chloroacetonitrile" SYNTHESIS, GEORG THIEME VERLAG, STUTTGART, DE, no. 12, 1 January 2000 (2000-01-01), pages 1709-1712, XP002457261 ISSN: 0039-7881 cited in the application
- DANYSZ W ET AL: "AMINO-ALKYL-CYCLOHEXANS AS A NOVEL CLASS OF UNCOMPETITIVE NMDA RECEPTOR ANTAGONISTS" CURRENT PHARMACEUTICAL DESIGN, BENTHAM SCIENCE PUBLISHERS, NL, vol. 8, no. 10, 1 January 2002 (2002-01-01), pages 835-843, XP008030349 ISSN: 1381-6128 cited in the application

## Description

### FIELD OF THE INVENTION

The present invention relates to a method of preparing 1-amino-1,3,3,5,5-pentamethylcyclohexane (Neramexane) or a pharmaceutically acceptable salt thereof.

### BACKGROUND OF THE INVENTION

1-amino-1,3,3,5,5-pentamethylcyclohexane (Neramexane) and pharmaceutically acceptable salts thereof are valuable agents for the continuous therapy of patients suffering from diseases and conditions such as tinnitus, and nystagmus.

Methods of preparing these agents are already known.

In one method, commercially available isophorone is converted to Neramexane in a reaction sequence comprising five steps according to the following reaction scheme (W. Danysz et al., Current Pharmaceutical Design, 2002, 8, 835-843):

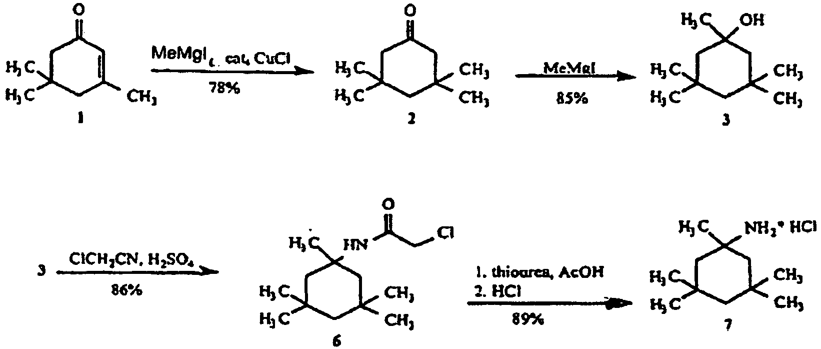

In the first step of the sequence, isophorone 1 is converted to 3,3,5,5-tetramethylcyclohexanone 2 by CuCl-catalyzed conjugate addition of methylmagnesium iodide.

In the second step, 3,3,5,5-tetramethylcyclohexanone 2 is converted to 1,3,3,5,5-pentamethylcyclohexanol 3 by Grignard reaction with methylmagnesium iodide.

In the third step, said cyclohexanol 3 is converted to 1-chloroacetamido-1,3,3,5,5-pentamethylcyclohexane 6 by chloroacetonitrile in a Ritter reaction.

In the subsequent fourth step, cleavage of the chloroacetamido group in amide 6 with thiourea in acetic acid, and acidification of the resulting amine with hydrochloric acid in the final fifth step of the reaction sequence results in Neramexane (1-amino-1,3,3,5,5-pentamethylcyclohexane) 7 in the form of its hydrochloride.

The cleavage of the chloroacetamido group in amide 6 has also been extensively investigated by Jirgensons et al. (Synthesis 2000, No. 12, 1709 - 1712). Accordingly, 1-chloroacetamido-1,3,3,5,5-pentamethylcyclohexane has been refluxed in a 5 : 1 mixture of ethanol and acetic acid. After a reaction time of 10 h, the reaction mixture has been diluted with water and the resulting precipitate has been isolated. The filtrate has been made alkaline and has been extracted with hexane. After addition of hydrochloric acid, 1-amino-1,3,3,5,5-pentamethylcyclohexane in the form of its hydrochloride has been isolated in a yield of 89 % by weight.

### OBJECTS OF THE INVENTION

One object of the invention is to improve one or more of the individual reaction steps of the above referenced reaction sequence in order to provide a method of preparing 1-amino-1,3,3,5,5-pentamethylcyclohexane or a pharmaceutically acceptable salt thereof that allows an advantageous realization on an economical industrial scale. It is in another object to minimize the amount of waste and/or unused chemicals produced during the manufacture of Neramexane or a pharmaceutically acceptable salt thereof. It is a further object to optimize or improve the yield and/or selectivity and/or product quality in regard to Neramexane or a pharmaceutically acceptable salt thereof. Particularly, the subject application aims to improve above step (iv), i.e. the reaction of 1-chloroacetamido-1,3,3,5,5-pentamethylcyclohexane with thiourea. Such an improved method may be regarded as one prerequisite for an advantageous manufacture of Neramexane or a pharmaceutically acceptable salt thereof on an economical industrial scale.

### SUMMARY OF THE INVENTION

The present invention relates to a method of preparing 1-amino-1,3,3,5,5-pentamethylcyclohexane comprising step (iv):
(iv) reacting a mixture comprising 1-chloroacetamido-1,3,3,5,5-pentamethylcyclohexane, thiourea and water.

In one embodiment, the mixture is substantially free from an organic solvent.

In one embodiment, the weight ratio of thiourea to water is in the range of from 1 : 0.5 to 1 : 50.

In another embodiment, the weight ratio of thiourea to water is in the range of from 1 : 1 to 1 : 20.

In another embodiment, the weight ratio of thiourea to water is in the range of from 1 : 2 to 1 : 10.

In one embodiment, the mixture further comprises an acid.

In one embodiment, the mixture comprises an acid in an amount of from 0.1 to 20 % by weight based on the amount of water.

In one embodiment, the acid is hydrochloric acid.

In one embodiment, the mixture is heated up to a temperature in the range of from 50 °C to the reflux temperature of the mixture.

In one embodiment, the mixture is heated up to a temperature in the range of from 80 °C to the reflux temperature of the mixture.

In one embodiment, per 1 mole 1-chloroacetamido-1,3,3,5,5-pentamethylcyclohexane, 1.0 to 2 mole thiourea, 1 to 3 mole acid and from 500 to 1,500 % by weight water based on the amount of thiourea and 1-chloroacetamido-1,3,3,5,5-pentamethylcyclohexane are employed at reflux temperature.

In one embodiment, alkali is added, after the mixture had been heated, in order to set the pH to a value of at least 7, and 1-amino-1,3,3,5,5-pentamethylcyclohexane is separated off from the mixture.

In one embodiment, the method further comprises step (v):
(v) adding an acid to 1-amino-1,3,3,5,5-pentamethylcyclohexane obtained in step (iv).

In one embodiment, the acid is methane sulphonic acid.

It has unexpectedly been discovered that the method according to the invention considerably shortens the reaction time as compared to the reaction time as disclosed in the methods of the prior art. It further considerably simplifies the workup of the amine to be produced, since an addition of water and filtration of a precipitate as referenced in the Background section is not necessary. The yield of amine is high and nearly quantitative. Thus, the novel method may be advantageously performed on an economical industrial scale.

### DETAILED DESCRIPTION OF THE INVENTION

The invention relates to a method of preparing 1-amino-1,3,3,5,5-pentamethylcyclohexane comprising step (iv):
(iv) reacting a mixture comprising 1-chloroacetamido-1,3,3,5,5-pentamethylcyclohexane, thiourea and water.

In one embodiment, the mixture employed in step (iv) further comprises an organic solvent.

In one embodiment, said organic solvent is a solvent that is miscible with water under the reaction conditions employed in step (iv), such as an alcohol.

In one embodiment, said organic solvent is an alcohol selected from the group consisting of methanol, ethanol, propanol, butanol, ethylene glycol.

In one embodiment, the amount of said organic solvent is from 0 to 200 % by weight based on the amount of water. In another embodiment, the amount of said organic solvent is from 0 to 150 % by weight, or from 0 to 100 % by weight, or from 0 to 50 % by weight, or from 0 to 10 % by weight, or from 0 to 5 % by weight based on the amount of water.

The mixture contains said organic solvent in an amount of from 0 to 5 % by weight based on the amount of water, or from 0 to 3 % by weight, or from 0 to 1 % by weight.

In one embodiment, the weight ratio of thiourea to water is in the range of from 1 : 0.5 to 1 : 50, or from 1 : 1 to 1 : 20, or from 1 : 2 to 1 : 10.

Although the reaction according to step (iv) may be performed without the addition of an acid, the addition thereof may accelerate the conversion of 1-chloroacetamido-1,3,3,5,5-pentamethylcyclohexane to 1-amino-1,3,3,5,5-pentamethylcyclohexane.

Accordingly, in one embodiment, the mixture of step (iv) further comprises an acid.

Acids that may be employed are, but are not limited to, hydrochloric acid, sulphuric acid, phosphorus acid, p-toluenesulphonic acid, methane sulphonic acid, acetic acid, benzoic acid. Accordingly, inorganic as well as organic acids may be used.

In one embodiment, the mixture in step (iv) contains no acetic acid.

The amount of acid employed, if any, may be in a relatively broad range.

In one embodiment, the mixture in step (iv) comprises an acid in an amount of from 0.1 to 20 % by weight based on the amount of water.

In one embodiment, the acid employed is hydrochloric acid.

In order to further accelerate the conversion of 1-chloroacetamido-1,3,3,5,5-pentamethylcyclohexane, the mixture employed in step (iv) is heated.

The term *"heating"* envisions that the mixture employed in step (iv) is set to a temperature above ambient temperature (25 °C).

In one embodiment, the mixture as employed in step (iv) is heated up to a temperature in the range of from 50 °C to the reflux temperature of the mixture.

In another embodiment, the mixture is heated up to a temperature in the range of from 80 °C to the reflux temperature of the mixture.

In still another embodiment, the mixture is heated up to the reflux temperature of the mixture.

If in step (iv) a mixture is employed that is substantially free from an organic solvent, the reflux temperature usually is around 100 °C, i.e. in the range of from 95 to 105 °C. If in step (iv) a mixture is employed that contains an organic solvent, the reflux temperature may be higher or lower than the reflux temperature of a mixture comprising water but that is substantially free from an organic solvent, depending on the amount and boiling point of the organic solvent employed.

The conversion of 1-chloroacetamido-1,3,3,5,5-pentamethylcyclohexane to 1-amino-1,3,3,5,5-pentamethylcyclohexane according to step (iv) may be controlled by the common chromatographical methods, e.g. by gas-liquid chromatography.

In one embodiment, in step (iv), per 1 mole 1-chloroacetamido-1,3,3,5,5-pentamethylcyclohexane 1.0 to 2 mole thiourea, 1 to 3 mole acid and 500 to 1,500 % by weight water based on the amount of thiourea and 1-chloroacetamido-1,3,3,5,5-pentamethylcyclohexane are employed at reflux temperature.

In one embodiment, the conversion is terminated already after 6 hours, or 5 hours, or even four hours, or even 3 hours, or even less than 3 hours.

In one embodiment, said 1-choroacetamido-1,3,3,5,5-pentamethylcyclohexane is reacted with approximately 1.2 molar equivalents thiourea and 2 molar equivalents hydrochloric acid in the 8-fold amount of water (by weight based on thiourea and 1-choroacetamido-1,3,3,5,5-pentamethylcyclohexane) at reflux temperature.

Commonly, the conversion in the water-containing mixture of step (iv) proceeds rather fast.

In one embodiment, wherein step (iv) is performed in water, i.e. the mixture is substantially free from an organic solvent, and wherein the heating is performed at reflux temperature, i.e. at a temperature around 100 °C, and wherein an acid is added, the conversion may even be terminated after 2 hours, or even 1 hour.

If the conversion is catalyzed by an acid, at least a part of the generated amine, i.e. the 1-amino-1,3,3,5,5-pentamethylcyclohexane, will be dissolved in water due to the protonation of the amino group, thus forming a salt.

In one embodiment, in order to isolate the produced amine, the method of the invention further comprises the addition of alkali to the mixture to set the pH to a value of at least 7, and separating off 1-amino-1,3,3,5,5-pentamethylcyclohexane from the mixture.

In said embodiment, preferably after cooling the mixture, the amine separates from the aqueous phase after the addition of alkali, and may be separated off.

In another embodiment, the amine may be extracted from the mixture which, after the addition of alkali, comprises an aqueous and an organic phase, with an organic solvent, which is not miscible with water. Suitable solvents are solvents such as methylene chloride, toluene or petroleum ether. Subsequent to the extraction, the extract may be dried using sodium sulphate or the like. After removing the solvent by evaporation, the crude amine is obtained.

In one embodiment, the yield of crude product is approximately better than 95 % of the theory (by weight), or even nearly quantitative. The crude product in general contains the target compound in a very high amount of at least 95 % by weight, or at least 97 % by weight, or even at least 99 % by weight as determined by gas-liquid chromatography.

In one embodiment, if necessary, the crude amine may be further purified by distillation.

*Conversion of 1-amino-1,3,3,5,5-pentamethylcyclohexane to a salt of 1-amino-1,3,3,5,5-pentamethylcyclohexane (step (v))*

In a subsequent step, 1-amino-1,3,3,5,5-pentamethylcyclohexane may be converted into a salt thereof by addition of an appropriate acid. In one embodiment, the salt is a pharmaceutically acceptable salt.

For the purpose of this disclosure, the term *"pharmaceutically acceptable* salts" refers to salts of neramexane that are physiologically tolerable and do not typically produce untoward reactions when administered to a mammal (e.g., human). Typically, the term *"pharmaceutically acceptable salt"* means approved by a regulatory agency of the Federal or a state government or listed in the U.S. Pharmacopeia or other generally recognized pharmacopeia for use in mammals, and more particularly in humans.

Conversion of 1-amino-1,3,3,5,5-pentamethylcyclohexane to a pharmaceutically acceptable salt thereof is accomplished in conventional fashion by admixture of the base with at least one molecular equivalent of a selected acid in an inert organic solvent. Isolation of the salt is carried out by techniques known to the art such as inducing precipitation with a non-polar solvent (e.g. ether) in which the salt has limited solubility. The nature of the salt is not critical, provided that it is non-toxic and does not substantially interfere with the desired pharmacological activity.

Examples of pharmaceutically acceptable salts are those formed with hydrochloric, hydrobromic, methanesulfonic, acetic, succinic, maleic, citric acid, and related acids.

Further pharmaceutically acceptable salts include, but are not limited to, acid addition salts, such as those made with hydroiodic, perchloric, sulfuric, nitric, phosphoric, propionic, glycolic, lactic, pyruvic, malonic, fumaric, tartaric, benzoic, carbonic, cinnamic, mandelic, ethanesulfonic, hydroxyethanesulfonic, benezenesulfonic, p-toluene sulfonic, cyclohexanesulfamic, salicyclic, p-aminosalicylic, 2-phenoxybenzoic, and 2-acetoxybenzoic acid.

In one embodiment, 1-amino-1,3,3,5,5-pentamethylcyclohexane as obtained and isolated in step (iv) is dissolved or dispersed or suspended in a solvent or a mixture of two or more of said solvents.

Suitable solvents are solvents such as acetone, anisole, butyl acetate, t-butylmethyl ether, cumene, dimethylsulphoxide, ethyl acetate, ethyl ether, ethyl formate, heptane, i-butyl acetate, i-propyl acetate, methyl acetate, methylethyl ketone, methyl-i-butyl ketone, pentane, propyl acetate, tetrahydrofurane, 1,1-diethoxypropane, 1,1-dimethoxymethane, 2,2-dimethoxypropane, isooctane, isopropyl ether, methyl-i-propyl ketone and methyltetrahydrofurane.

In one embodiment, mixtures of solvents and water such as methylethyl ketone and water may also be used.

Subsequent to the dissolving or dispersing or suspending, an appropriate acid is added in order to allow for the formation of the salt. Said acid may also be dissolved or dispersed or suspended in one or more of the above defined solvents.

The precipitated and/or crystallized salt may be separated off from the reaction mixture by filtration.

Solvent adhering to the precipitate may be removed by applying vacuum and /or heat.

In one embodiment, the employed acid is hydrochloric acid or methane sulphonic acid, and the salt is the chloride or the mesylate.

In one embodiment, methane sulphonic acid is added to 1-amino-1,3,3,5,5-pentamethylcyclohexane.

In one embodiment, the yield of salt is better than 95 % by weight having a purity of more than 99.9 % by weight.

### EXAMPLES

### Example 1

A mixture of 245 g 1-chloroacetamido-1,3,3,5,5-pentamethylcyclohexane, 91 g thiourea, 2,700 g water and 220 g hydrochloric acid (33 % acid) is heated under reflux. After a reaction time of 6 hours, the mixture is cooled to ambient temperature, and the pH of the mixture is set to a value of greater than 7 by adding sodium hydroxide. Subsequently, the mixture is extracted twice with petroleum ether. The extracts are combined. After distilling petroleum ether off, 159 g crude 1-amino-1,3,3,5,5-pentamethylcyclohexane is obtained (97 % yield). The crude product has a content of target compound of 97 % by weight as determined by gas-liquid chromatography. Subsequent, the crude product is distilled in order to further purify it.

### Example 2

Example 1 is repeated. The yield of crude target compound is 100 % having a content of 99 % by weight target compound.

## Claims

1. Method of preparing 1-amino-1,3,3,5,5-pentamethylcyclohexane or a pharmaceutically acceptable salt thereof comprising step (iv):
(iv) reacting a mixture comprising 1-chloroacetamido-1,3,3,5,5-pentamethylcyclohexane, thiourea and water.

2. Method according to claim 1, wherein the mixture comprises an organic solvent in an amount of from 0 to 5 % by weight based on the amount of water.

3. Method according to claim 1 or 2, wherein the weight ratio of thiourea to water is in the range of from 1 : 0.5 to 1 : 50.

4. Method according to any one of the preceding claims, wherein the weight ratio of thiourea to water is in the range of from 1 : 1 to 1 : 20.

5. Method according to any one of the preceding claims, wherein the weight ratio of thiourea to water is in the range of from 1 : 2 to 1 : 10.

6. Method according to any one of the preceding claims, wherein the mixture further comprises an acid.

7. Method according to claim 6, wherein the mixture comprises an acid in an amount of from 0.1 to 20 % by weight based on the amount of water.

8. Method according to claim 6 or 7, wherein the acid is hydrochloric acid.

9. Method according to any one of the preceding claims, wherein the mixture is heated up to a temperature in the range of from 50 °C to the reflux temperature of the mixture.

10. Method according to any one of the preceding claims, wherein the mixture is heated up to a temperature in the range of from 80 °C to the reflux temperature of the mixture.

11. Method according to any one of the preceding claims, wherein in step (iv) per 1 mole 1-chloroacetamido-1,3,3,5,5-pentamethylcyclohexane 1.0 to 2 mole thiourea, 1 to 3 mole acid and 500 to 1,500 % by weight water based on the amount of thiourea and 1-chloroacetamido-1,3,3,5,5-pentamethylcyclohexane are employed at reflux temperature.

12. Method according to any one of the preceding claims, further comprising the addition of alkali to the mixture to set the pH to a value of at least 7, and separating off 1-amino-1,3,3,5,5-pentamethylcyclohexane from the mixture.

13. Method according to any one of the preceding claims, further comprising step (v):
(v) adding an acid to 1-amino-1,3,3,5,5-pentamethylcyclohexane obtained in step (iv).

14. Method according to claim 13, wherein the acid is methane sulphonic acid.

## Patentansprüche

1. Verfahren zur Herstellung von 1-Amino-1,3,3,5,5-pentamethylcyclohexan oder eines pharmazeutisch verträglichen Salzes davon, umfassend Schritt (iv):
(iv) Reagieren einer Mischung umfassend 1-Chloracetamido-1,3,3,5,5-pentamethylcyclohexan, Thioharnstoff und Wasser.

2. Verfahren nach Anspruch 1, wobei die Mischung ein organisches Lösungsmittel in einer Menge von 0 bis 5 Gew.-% umfasst, bezogen auf die Menge an Wasser.

3. Verfahren nach Anspruch 1 oder 2, wobei das Gewichtsverhältnis von Thioharnstoff zu Wasser im Bereich von 1 : 0,5 bis 1 : 50 liegt.

4. Verfahren nach einem der vorstehenden Ansprüche, wobei das Gewichtsverhältnis von Thioharnstoff zu Wasser im Bereich von 1 : 1 zu 1 : 20 liegt.

5. Verfahren nach einem der vorstehenden Ansprüche, wobei das Gewichtsverhältnis von Thioharnstoff zu Wasser im Bereich von 1 : 2 zu 1 : 10 liegt.

6. Verfahren nach einem der vorstehenden Ansprüche, wobei die Mischung weiter eine Säure umfasst.

7. Verfahren nach Anspruch 6, wobei die Mischung eine Säure in einer Menge von 0,1 bis 20 Gew.-% umfasst, bezogen auf die Menge an Wasser.

8. Verfahren nach Anspruch 6 oder 7, wobei die Säure Salzsäure ist.

9. Verfahren nach einem der vorstehenden Ansprüche, wobei die Mischung bis zu einer Temperatur im Bereich von 50 °C bis zur Rückflusstemperatur der Mischung erhitzt wird.

10. Verfahren nach einem der vorstehenden Ansprüche, wobei die Mischung bis zu einer Temperatur im Bereich von 80 °C bis zur Rückflusstemperatur der Mischung erhitzt wird.

11. Verfahren nach einem der vorstehenden Ansprüche, wobei in Stufe (iv) pro 1 Mol 1-Chloracetamido-1,3,3,5,5-pentamethylcyclohexan 1,0 bis 2 Mol Thioharnstoff, 1 bis 3 Mol Säure und 500 bis 1500 Gew.-% Wasser bei Rückflusstemperatur verwendet werden, bezogen auf die Menge an Thioharnstoff und 1-Chloraceteamido-1,3,3,5,5-pentamethylcyclohexan.

12. Verfahren nach einem der vorstehenden Ansprüche, weiter umfassend das Hinzufügen von Alkali zur Mischung, um den pH auf einen Wert von mindestens 7 einzustellen, und um 1-Amino-1,3,3,5,5-pentamethylcyclohexan aus der Mischung abzutrennen.

13. Verfahren nach einem der vorstehenden Ansprüche, weiter umfassend Stufe (v):
(v) Hinzufügen einer Säure zum 1-Amino-1,3,3,5,5-pentamethylcyclohexan, welches in Stufe (iv) erhalten wurde.

14. Verfahren nach Anspruch 13, wobei die Säure Methansulfonsäure ist.

## Revendications

1. Procédé de préparation du 1-amino-1,3,3,5,5-pentaméthyl-cyclohexane ou d'un sel pharmaceutiquement acceptable de celui-ci comprenant l'étape (iv) :
(iv) réaction d'un mélange comprenant du 1-chloroacétamido-1,3,3,5,5-pentaméthylcyclohexane, de la thiourée et de l'eau.

2. Procédé selon la revendication 1, où le mélange comprend un solvant organique en une quantité de 0 à 5 % en poids sur la base de la quantité d'eau.

3. Procédé selon la revendication 1 ou 2, où le rapport en poids de la thiourée à l'eau est dans la plage de 1 : 0,5 à 1 : 50.

4. Procédé selon l'une quelconque des revendications précédentes, où le rapport en poids de la thiourée à l'eau est dans la plage de 1 : 1 à 1 : 20.

5. Procédé selon l'une quelconque des revendications précédentes, où le rapport en poids de la thiourée à l'eau est dans la plage de 1 : 2 à 1 : 10.

6. Procédé selon l'une quelconque des revendications précédentes, où le mélange comprend en outre un acide.

7. Procédé selon la revendication 6, où le mélange comprend un acide en une quantité de 0,1 à 20 % en poids sur la base de la quantité d'eau.

8. Procédé selon la revendication 6 ou 7, où l'acide est l'acide chlorhydrique.

9. Procédé selon l'une quelconque des revendications précédentes, où le mélange est chauffé jusqu'à une température dans la plage de 50°C à la température de reflux du mélange.

10. Procédé selon l'une quelconque des revendications précédentes, où le mélange est chauffé jusqu'à une température dans la plage de 80°C à la température de reflux du mélange.

11. Procédé selon l'une quelconque des revendications précédentes où, dans l'étape (iv), par mole de 1-chloroacétamido-1,3,3,5,5-pentaméthylcyclohexane 1,0 à 2 moles de thiourée, 1 à 3 moles d'acide et 500 à 1 500 % en poids d'eau sur la base de la quantité de thiourée et de 1-chloroacétamido-1,3,3,5,5-pentaméthylcyclohexane sont employées à la température de reflux.

12. Procédé selon l'une quelconque des revendications précédentes, comprenant en outre l'addition d'un alcali au mélange pour fixer le pH à une valeur d'au moins 7, et la séparation du 1-amino-1,3,3,5,5-pentaméthylcyclohexane d'avec le mélange.

13. Procédé selon l'une quelconque des revendications précédentes, comprenant en outre l'étape (v) :
(v) addition d'un acide au 1-amino-1,3,3,5,5-pentaméthylcyclohexane obtenu dans l'étape (iv).

14. Procédé selon la revendication 13, où l'acide est l'acide méthanesulfonique.
